# EUROPEAN PATENT APPLICATION

(11) **EP 4 331 639 A1**
(43) Date of publication of application: **06.03.2024**
(21) Application number: 22731786.4
(22) Date of filing: 28.04.2022
(51) Int. Cl.: A61M 1/34, A61K 38/38, A61P 25/28, A61M 1/36

(54) **USE OF THERAPEUTIC PLASMA EXCHANGE AND LOW VOLUME PLASMA EXCHANGE FOR THE TREATMENT OF A COGNITIVE IMPAIRMENT**

(30) Priority: 30.04.2021 EP 21382388
(71) Applicant: Grifols Worldwide Operations Limited, Dublin 22 (IE)
(72) Inventor: GRIFOLS ROURA, Victor, 08174 Sant Cugat Del Valles ( Barcelona) (ES); PAEZ REGADERA, Antonio Manuel, 08174 Sant Cugat Del Valles ( Barcelona) (ES); NUÑEZ DOMENECH, Laura, 08174 Sant Cugat Del Valles ( Barcelona) (ES)
(74) Representative: Durán-Corretjer, S.L.P.
(86) International application number: PCT/IB2022/000246
(87) International publication number: WO 2022/229705

(57) **Abstract**

The present invention refers to a composition comprising human albumin at a concentration between 5 % (w/v) and 25 % (w/v) for the treatment of a cognitive impairment by conventional therapeutic plasma exchange (TPE) and low-volume plasma exchange (LVPE) in a patient in need thereof, wherein said patient has a Mini Mental State Examination (MMSE) greater or equal to 15.

## Description

The present invention relates to the medical field, in particular to the use of a therapy combining therapeutic plasma exchange (TPE) and low volume plasma exchange (LVPE) for the treatment of a cognitive impairment, wherein plasma is replaced with a solution comprising albumin.

Recently, the present inventors have developed a treatment combining TPE and LVPE specific for mild and moderate Alzheimer's Disease (AD) (WO 2020/084346 A1). Now, it has been discovered that a similar treatment but using different approach is useful for the treatment of other cognitive impairments, such as for example Lewy Body Dementia (LBD), Parkinson's Disease (PD), and Mild Cognitive impairment (MCI).

LBD and PD are very well known diseases. However, MCI is a concept recently proposed by scientists. MCI or mild neurocognitive disorder is an intermediate state (Petersen, RC, Lopez, O, Armstrong, MJ. (2018) Neurology;90(3):126-135) between normal aging and dementia. This state can progress to dementia, mostly in the form of Alzheimer's disease (Lopez, OL (2013) Continuum (Minneap Minn);19(Dementia):411-424).

The prevalence of MCI in adults older than 60 is approximately 6.7 % to 25.2 %. It increases with age and lower level of education, and is more prevalent in men than in women (Petersen, RC, et al., (2018) Neurology; 90(3):126-135).

Formerly, MCI was defined by focusing mainly on amnesia, but it later includes a wider definition that covers either impairment in single-domain nonamnestic or several cognitive domains with or without memory deficit. The annual rate of progression to dementia is approximately 5 % to 17 % (Ganguli, M, et al., (2013) Neurology;80(23):2112-2120). Some established biomarkers associated with the progression from MCI to Alzheimer's disease are a positive amyloid positron emission tomography (PET) scan, apolipoprotein E4 genotype, abnormal cerebrospinal fluid (CSF) tau levels, a positive PET scan due to tau deposition into the lateral temporal lobe structures (Cheng, YW, et al., (2017) Neuropsychiatr Dis Treat;13:491-498).

Unfortunately, currently, there are no medications approved by the Food and Drug Administration (FDA) for the treatment of MCI. Drugs approved to treat symptoms of Alzheimer's disease have not shown any lasting benefit in delaying or preventing progression of MCI to dementia.

Identifying cognitive dysfunction in these individuals as early as possible is desirable so that appropriate treatment strategies can be implemented earlier in the course of the disease.

Therefore, there is still a need of successful treatments of cognitive impairment in a patient in need thereof. The present inventors have surprisingly found that using albumin as replacement fluid combining TPE and LVPE helps to prevent or slow cognitive decline.

The treatment regime of LVPE requires smaller volumes than traditional therapeutic plasma exchange, shorter duration time and a single venipuncture, decreasing patient discomfort, and reducing adverse effects of the treatment.

In a first aspect, the present invention refers to the use of human albumin at a concentration between 5 % (w/v) and 25 % (w/v) for the treatment of a cognitive impairment in a patient in need thereof, wherein said treatment comprises a combination of conventional therapeutic plasma exchange (TPE) and low-volume plasma exchange (LVPE), and wherein said patient has a Mini Mental State Examination (MMSE) greater or equal to 15.

As used herein, the term "Mini Mental State Examination (MMSE)" refers to a score used to screen patients for cognitive impairment, track changes in cognitive functioning over time, and oftentimes to assess the effects of therapeutic agents on cognitive function (Folstein MF, et al., (1975) J Psychiatr Res.;12:189 98). MMSE is the most commonly administered psychometric screening assessment of cognitive functioning.

As used herein, the term "low-volume plasma exchange" or "LVPE" refers to plasma exchange treatments involving blood volumes between 600 mL - 900 mL.

According to particular embodiments, each round of LVPE involves automated plasma exchange using a single venipuncture. Any extracorporeal plasma exchange device known in the art may be used in accordance with the present invention. For example, the device may include an entrance port for receiving the whole blood from a patient, means for separating plasma from the cellular components of the blood, and a means for returning the cellular components of the blood through the entrance port from where the blood cellular components exited the device, and an exit port from where the plasma exits the device. Preferably, the means for separating plasma from the cellular components of the blood are centrifugation means (i.e., a centrifuge). More preferably, the means for separating plasma from the cellular components of the blood are filtration means (i.e., a filter such as that used in double filtration plasma exchange).

According to particular embodiments, the treatment regime comprises administering three or more rounds of LVPE, four or more rounds of LVPE, five or more rounds of LVPE, six or more rounds of LVPE, seven or more rounds of LVPE, eight or more rounds of LVPE, nine or more rounds of LVPE, or ten or more rounds of LVPE, eleven or more rounds of LVPE, or twelve or more rounds of LVPE to the patient.

According to particular embodiments, each subsequent round of LVPE is conducted 10-45 days after the previous round. Preferably, each subsequent round of LVPE is conducted 15-35 days after the previous round, more preferably conducted 30 days after the previous round.

In one embodiment, said LVPE is conducted at a frequency of 1 LVPE per month.

In one embodiment, said monthly LVPE is conducted at least during 12 months.

In one embodiment, said second stage of LVPE is conducted chronically as long as patient shows response.

According to particular embodiments, between 20 g and 50 g of albumin can be used for replacement in each round of LVPE, preferably 40 g of albumin in each round of LVPE.

According to particular embodiments, in said LVPE regime the plasma is replaced with albumin at a concentration between 18 % (w/v) and 25 % (w/v), preferably at 20 % (w/v).

In another particular embodiment, before the treatment regime of LVPE previous rounds of conventional therapeutic plasma exchange (TPE) can be carried out. Preferably, said TPE regime can be conducted at a frequency of 1 TPE per week, more preferably at least during 6 weeks. The term "TPE" refers to a plasma exchange in which 2000 to 3000 mL of patient's plasma was replaced with the same volume of a replacement fluid.

According to particular embodiments, between 100 and 150 g of albumin can be used for replacement in each round of TPE.

According to particular embodiments, in said TPE regime the plasma is replaced with albumin at a concentration between 4 % (w/v) and 6 % (w/v), preferably at 5 % (w/v).

In one embodiment, said cognitive impairment is selected from the list comprising Alzheimer's Disease (AD), Lewy Body Dementia (LBD), Parkinson's Disease (PD), and Mild Cognitive impairment (MCI), preferably said cognitive impairment is selected from Alzheimer's Disease (AD) and Mild Cognitive impairment (MCI), more preferably said cognitive impairment is Mild Cognitive impairment (MCI).

In one embodiment, the patient in need of the treatment of the present invention must fulfill the following eligibility criteria:
- Negative PCR for COVID-19 or vaccinated for SARS-CoV-2.
- Age between 50 and 85 years old.
- Nuclear Magnetic Resonance (NMR) of the brain in the last 6 months, to rule out relevant brain pathology (Neurology).
- Absence of neurologic or psychiatric disease.
- Absence of known hematologic disease.
- Patients with anticoagulant therapy that should be individually assessed to modify, if possible, the anticoagulant regimen or rule out those with a history of hemorrhagic diathesis at risk of bleeding during the procedure.
- Absence of significant hepatic or renal disease.
- Absence of active acute infections.
- Absence of class III and IV heart failure (classification according to the New York Heart Association):
   a. Class III: Marked limitation of the physical activity. Comfortable at rest. Physical activity less than ordinary causes fatigue, palpitations, dyspnea or anginal pain.
   b. Class IV: Unable to carry out any physical activity without discomfort. Presence of symptoms of angina or heart failure even at rest.
- Absence of uncontrolled blood hypertension.
- Absence of diabetes or other decompensated metabolic disease.
- Absence of rhythm disorders or cardiac conduction with risk of sudden death.
- Absence of Chronic Obstructive Pulmonary Disease (COPD) in need of oxygen or chronic therapy.
- Good venous access for conducting the therapeutic apheresis.
- Possibility of assisting to the therapy.
- Absence of renal, hepatic, cardiac or lung transplantation requiring immunosuppression with Cyclosporine or similar drugs susceptible to be altered with plasmapheresis.
- Absence of allergy human serum albumin.

### Example 1. Medical assistance protocol

The reason for this medical assistance protocol is to ensure the best care and treatment of candidate patients to receive the AMBAR treatment.

AMBAR treatment consists of two periods of therapeutic apheresis with the following recommendation of the Treatment Supervisory Committee (TSC):
- A weekly therapeutic plasma replacement (TPE) procedure will be conducted with an initial guideline of 6 weeks of TPE. A plasmatic volume exchange with be performed with replacement of between 125 and 150 g of Albutein^{®} 5 % or Plasbumin^{®} 5 %.
- Once completed the TPE period, continuation with low volume plasma exchange (LVPE) with a frequency of 1 LVPE monthly will be evaluated, according to the TSC criteria. LVPE consists of exchange of between 690 and 880 ml of plasma with reposition of 40 g of Albutein^{®} 20 % or Plasbumin^{®} 20 %.

The medical assistance protocol is divided into four phases: selection phase, prescription phase, application phase, and follow-up phase.

### Selection Phase

The aim of the selection phase is to evaluate all the medical information available from the patient and, if necessary, request further information, in order to evaluate suitability of the patient in the next phase, the prescription phase.

The selection phase comprises a first visit Neurology/Internal medicine, and different complementary visits.

### Eligibility Criteria

A patient candidate for AMBAR treatment must meet the eligibility criteria described below. Eligibility criteria should be assessed during the first visit of Neurology/Internal Medicine supplemented with analysis needed to make a decision on the second visit of Neurology/Internal Medicine, on whether or not said candidate patient is eligible or non-eligible for the AMBAR treatment.

The evaluation of the criteria is performed by both the Neurologist and the Internist, being each one responsible for compliance with criteria (between parenthesis the responsible of conformity of each criterion is indicated). At the time of deciding if the patient is eligible or non-eligible, both professionals must give their agreement.
- Negative PCR for COVID-19 or vaccinated for SARS-CoV-2 (Neurology).
- Signing information sheet of the patient and informed consent by the patient, family member or legal guardian (Neurology/Internal Medicine).
- Presence of cognitive impairment single or multiple domain with basal Mini Mental Status Examination (MMSE) ≥ 15 (Neurology).
- Age between 50 and 85 years old (Neurology).
- Nuclear Magnetic Resonance (NMR) of the brain in the last 6 months, to rule out relevant brain pathology (Neurology).
- Absence of neurologic or psychiatric disease which according to the physicians responsible for the treatment prevents conducting of the same.
- Absence of known hematologic disease that according to TSC criteria poses the patient at risk.
- Patients with anticoagulant therapy that should be individually assessed by the TSC to modify, if possible, the anticoagulant regimen or rule out those with a history of hemorrhagic diathesis at risk of bleeding during the procedure (Internal Medicine).
- Absence of significant hepatic or renal disease that the TSC considers at risk.
- Absence of active acute infections (Internal Medicine).
- Absence of class III and IV heart failure (classification according to the New York Heart Association): (Internal Medicine)
   a. Class III: Marked limitation of the physical activity. Comfortable at rest. Physical activity less than ordinary causes fatigue, palpitations, dyspnea or anginal pain.
   b. Class IV: Unable to carry out any physical activity without discomfort. Presence of symptoms of angina or heart failure even at rest.
- Absence of uncontrolled blood hypertension. Treatment with beta-blockers that determine a cardiac frequency < 60 bpm (Internal Medicine).
- Absence of diabetes or other decompensated metabolic disease (Internal Medicine).
- Absence of rhythm disorders or cardiac conduction with risk of sudden death.
- Absence of Chronic Obstructive Pulmonary Disease (COPD) in need of oxygen or chronic therapy (Internal Medicine).
- Good venous access for conducting the therapeutic apheresis (Internal Medicine).
- Possibility of assisting to visits and tests defined in this protocol (Neurology/Internal Medicine).
- Absence of renal, hepatic, cardiac or lung transplantation requiring immunosuppression with Cyclosporine or similar drugs susceptible to be altered with plasmapheresis (Internal Medicine).
- Absence of allergy human serum albumin (Internal Medicine).

### Discontinuity Criteria

Throughout the AMBAR treatment there are defined discontinuity reasons of a patient, always under supervision and criteria of TSC. Specifically, a patient will end its participation in the AMBAR medical assistance protocol:
1. Developing an inadequate venous access that cannot be mitigated and prevents performing of the therapeutic apheresis.
2. Occurrence of pathology incompatible with the treatment.
3. Progression greater than expected of the cognitive and functional impairment.
4. Withdrawal of consent by the patient.

### First visit of Neurology and Internal Medicine

Patients eligible for the AMBAR treatment will be cited to facilities for a first visit for a Neurology and Internal Medicine evaluation.

The prerequisites for the visit are that the patient is discharged by administration with consequent assignation of a single AMBAR code. Also the signing of the general consent of personal data protection and assignment will be obtained.

Given the situation or current pandemic, patients must have a negative PCR for COVID-19 to be able to participate and attend in person the different visits that the AMBAR treatment requires. Also it is appropriate to ask whether or not the patient has been vaccinated against SARS-COV-2.

The procedures to be performed during the first visit of Neurology/Internal Medicine are:
Neurology
   - Obtaining the general informed consent AMBAR, consent for obtaining biological samples for future research and consent for obtaining genetic samples.
   - Collecting previous clinic history and performing neurological exploration. An overall medical evaluation has to be made and knowing the neurological history of the patient, including medication that the patient receives.
   - Perform MMSE and CDR tests.
   - Assessing of previous nuclear magnetic resonance (NMR) tests if applicable, and in case that no NMR test is available, requesting a supplementary visit (see section below)
   - Reviewing suitability criteria.
   - Asking about allergies and get a list of previous medication.
   - Generating report of the visit and requesting additional tests (neuropsychology visit, etc.).
Nursing
   - Obtaining nursing data (weight, height, body mass index, vital signs, electrocardiogram [ECG]).
   - Assessing venous access - determining how many puncture points in the left and right arms are compatible with the therapeutic apheresis and gauge size of the venous access to be used.
Internal Medicine
   - Confirm obtaining the general informed consent AMBAR, consent for obtaining biological samples for future research and consent for obtaining genetic samples.
   - Asking about allergies and obtaining a list of previous medication.
   - Internal Medicine exploration about health status.
   - Generating a visit report and requesting additional tests.

### Complementary visits

Complementary visits aim to obtain additional information to complete the first visit of Neurology/Internal Medicine. Complementary visits include both clinic visits as well as visits for additional tests, which can be performed outside of the center, and will be the following:
Neuropsychological and functional assessment
One neuropsychological exploration will be performed by a qualified professional.

### Blood test

Blood analysis will be performed to assess the hematologic function, biochemistry and clotting of the patient in order to know if the patient meets the eligibility criteria. Blood analysis should be performed at most 1 week before the first TPE treatment and results should be available before the second visit (Neurology/Internal Medicine) (Eligible/Non eligible)
∘ Asking if the patient is fasting. This is a relevant question to biological samples obtained in the visit.
∘ Hematology

- Complete blood count
   ∘ Red blood cells
   ∘ Hematocrit
   ∘ Hemoglobin
   ∘ Mean corpuscular volume (MCV)
   ∘ Mean corpuscular hemoglobin (HCM)
   ∘ Leukocyte
   ∘ Neutrophils
   ∘ Lymphocytes
   ∘ Monocytes
   ∘ Eosinophils
   ∘ Basophils
   ∘ Platelets
- Erythrocyte sedimentation rate (ESR)
   ∘ Biochemistry
      ∘ Ferritin
      ∘ Total proteins
      ∘ Albumin
      ∘ Globulin
      ∘ Glycemia
      ∘ Urea
      ∘ Creatinine
      ∘ Sodium
      ∘ Potassium
      ∘ Calcium
      ∘ Chloride
      ∘ Magnesium
   ∘ Clotting
      ∘ Fibrinogen
      ∘ Quick Time / Prothrombin Time (TP)
      ∘ Cephalin Time / Activated Partial Thromboplastin Time (aPTT)
      ∘ International Normalized ratio (INR)
   ∘ Immunology
      ∘ Immunoglobulin G (IgG)
      ∘ Immunoglobulin A (IgA)
      ∘ Immunoglobulin M (IgM)
      ∘ Anti-thyroid antibodies
      ∘ Human immunodeficiency virus (HIV) serology
      ∘ Hepatitis C virus (HCV) serology
   ∘ Hepatic enzymes
      ∘ Aspartate aminotransferase (AST / GOT)
      ∘ Alanine aminotransferase (ALT / GPT)
      ∘ Alkaline phosphatase
      ∘ Gamma-glutamyl transferase (GGT)
   ∘ Thyroid tests
      ∘ T3
      ∘ T4
      ∘ free T4
      ∘ TSH
   ∘ Urine
      ∘ Sediment

### Brain Nuclear Magnetic Resonance (NMR)

in order to meet the criterion of suitability "Nuclear Magnetic Resonance of the brain in the last 6 months to rule out relevant brain pathology", the patient should present during the 1st visit of Neurology/Internal Medicine a radiological report of previous NMR tests. If this is not possible, between the 1st visit of Neurology/Internal Medicine and the 2nd visit of Neurology/Internal Medicine, a NMR of the brain will be requested to the patient.

In this additional visit to check the NMR test it should be indicated that in the radiology report the following scales has to be assessed:
- Global cortical atrophy scale (Pasquier)
- Parietal atrophy scale (Koedam)
- Medial temporal atrophy scale (Scheltens)
- Vascular Scale (Fazekas)
- Presence and quantity of Microbleeds and superficial siderosis

If it is not possible, the two more important scales to register are Scheltens and Fazekas.

### Prescription Phase

The aim of the prescription phase is to decide whether the patient is suitable or not to receive the AMBAR treatment, and if so, preparing the patient for the next phase, the application phase.

The prescription phase comprises a second visit of Neurology/Internal Medicine (Eligible / Non eligible).

### Second visit of Neurology and Internal Medicine

Patients eligible for the AMBAR treatment will be appointed in the facilities for a second visit of evaluation by Neurology and Internal Medicine.

The prerequisites for the visit are that the patient has already made the first visit by Neurology/Internal Medicine, and also all those necessary complementary visits to confirm eligibility criteria.

Given the situation or current pandemic, patients must have a negative PCR for COVID-19. Also it is appropriate to ask if the patient has been vaccinated against SARS-COV-2.

The procedures to be performed during the 2nd visit of Neurology/Internal Medicine are:
- Evaluating all the available information from the patient generated during the patient's first visit of Neurology/Internal Medicine and/or obtained in the supplementary visits.
- Assessing previous and current medication. Reset pattern of current therapy depending on the drug and dosing

- Conforming to the suitability criteria.
- Deciding whether the patient is suitable or unsuitable for the AMBAR medical treatment.
- If so, obtaining the informed consent of the patient to start treatment.
- Prescribing treatment of therapeutic apheresis AMBAR.
- Generating the treatment report prescribed for the reference physician and the patient.

### Application phase - Therapeutic plasma exchange (TPE)

### Before the TPE

### Nursing

Before any procedure of therapeutic plasma exchange (TPE) the nurse/apherist will explore the patient to verify that the patient may receive the treatment. This exploration can be done just before the treatment or at most 48 hours before. This exploration consists of:
- Asking the patient if he is fasting. The patient can receive treatment whether the patient is or not fasting. This question is relevant to the biological samples obtained from the visit.
- Recording the weight, height and blood volume, calculated by the apheresis machine in order to calculate the amount of plasma to be exchanged.
- Performing a blood analysis - Previous to TPE
   ∘ Hematology

   - Complete blood count
      ∘ Red blood cells
      ∘ Hematocrit
      ∘ Hemoglobin
      ∘ Mean corpuscular volume (MCV)
      ∘ Mean corpuscular hemoglobin (HCM)
      ∘ Leukocyte
      ∘ Neutrophils
      ∘ Lymphocytes
      ∘ Monocytes
      ∘ Eosinophils
      ∘ Basophils
      ∘ Platelets
   - Erythrocyte sedimentation rate (ESR)
      ∘ Biochemistry
         ∘ Total proteins
         ∘ Albumin
         ∘ Globulin
         ∘ Glycemia
         ∘ Urea
         ∘ Creatinine
         ∘ Sodium
         ∘ Potassium
         ∘ Calcium
         ∘ Chloride
         ∘ Magnesium
      ∘ Clotting
         ∘ Fibrinogen
         ∘ Quick Time / Prothrombin Time (TP)
         ∘ Cephalin Time / Activated Partial Thromboplastin Time (aPTT)
         ∘ International Normalized ratio (INR)
      ∘ Immunology
         ∘ Immunoglobulin G (IgG)
         ∘ Immunoglobulin A (IgA)
         ∘ Immunoglobulin M (IgM)
      ∘ Hepatic enzymes
         ∘ Aspartate aminotransferase (AST / GOT)
         ∘ Alanine aminotransferase (ALT / GPT)
         ∘ Alkaline phosphatase
         ∘ Gamma-glutamyl transferase (GGT)
      ∘ COVID-19
         ∘ PCR SARS-COV-2
      ∘ Urine
         ∘ Sediment
- Collect biological sample before procedure. It will be done at the same time as the extraction blood for analysis.
- Perform an electrocardiogram (ECG)
- Take vital signs - Pre LVPE
   ∘ Systolic and diastolic blood pressure
      ∘ Heart rate
      ∘ Respiratory rate
      ∘ Temperature
      ∘ Pulse oximetry to measure partial oxygen saturation

### internist

Then, the internist physician will make an assessment of the patient to determine whether or not the patient can receive the prescribed treatment. To do this, it must:
- Take an anamnesis of recent processes.
- Review the exploration made by the nurse.
- Assess potential new treatments started since the last visit.

### Starting the procedure

Once the internist physician has concluded that the patient is suitable for the treatment, the nurse / apherist will place 2 peripheral venous accesses to connect the patient to the TPE machine. The treatment following the AMBAR standard consists of 6 TPE with replacement of 125-150 g Albutein ^{®} 5 % or Plasbumin ^{®} 5 %. It has to be performed once a week for 6 weeks. This pattern can be modified according to the physician criterion.

It will be recorded on the data collection screens if the procedure has been started or not (and why it has not been started), time, in addition to all the information related with the machine, solutions and materials used for the TPE.

The TPE procedure will be done by the nurse under the supervision of the internist physician.

If the internist physician considers that the patient is not able to receive the treatment, the patient will go straight to the second visit of Neurology (TPE follow-up visit).

Should any situation occur that requires medical emergency to the patient, the established protocol should be consulted.

### During the TPE

### Nursing

During the TPE procedure the nurse/apherist will explore the patient to verify that the patient may continue receiving the treatment. This exploration can be done just before the treatment or at most 48 hours before. This exploration consists of:
- Asking the patient if he is fasting. The patient can receive treatment whether the patient is or not fasting. This question is relevant to the biological samples obtained from the visit.
- Performing a blood analysis - During TPE it will be optional at the discretion of the physician and can be performed further times depending of the patient's condition.
   ∘ Hematology
      ∘ Hemoglobin
   ∘ Biochemistry
      ∘ Glycemia
      ∘ Sodium
      ∘ Potassium
      ∘ Calcium
      ∘ Chloride
      ∘ Magnesium
   ∘ Acid/base equilibrium Clotting

   - Collect vital signs. During TPE, that will be taken 3 times during the TPE and should be recorded in the evaluation sheet.
      ∘ Systolic and diastolic blood pressure
      ∘ Heart rate
      ∘ Respiratory rate
      ∘ Temperature
      ∘ Pulse oximetry to measure partial oxygen saturation

Data will be collected related with the estimated blood volume of the patient calculated by the TPE machine. Also, data will be collected related to the TPE process every 30 minutes.

It will be evaluated and collected all the incidents and adverse events that may occur during the procedure either a problem with the machine, by a human error, problems with the medication or treatment or for any other reason. Any medication administered to the patient to solve said adverse event will be also recorded.

### After the TPE

Once the procedure is finished, the end time will be noted, as well as the duration of the procedure. It is understood as the duration from when the patient enters the center until he leaves it. It will also be collected if the procedure has been completed or not (and the reasons why it has not been completed). The nurse will do an exploration to the patient to verify the patient's condition after receiving treatment. This exploration will consist of:
- Asking if the patient is fasting. This is a relevant question to biological samples obtained in the visit.
- Analytical - Post TPE
   ∘ Hematology

   - Complete blood count
      ∘ Red blood cells
      ∘ Hematocrit
      ∘ Hemoglobin
      ∘ Mean corpuscular volume (MCV)
      ∘ Mean corpuscular hemoglobin (HCM)
      ∘ Leukocyte
      ∘ Neutrophils
      ∘ Lymphocytes
      ∘ Monocytes
      ∘ Eosinophils
      ∘ Basophils
      ∘ Platelets
   - Erythrocyte sedimentation rate (ESR)
      ∘ Biochemistry
         ∘ Total proteins
         ∘ Albumin
         ∘ Globulin
         ∘ Glycemia
         ∘ Urea
         ∘ Creatinine
         ∘ Sodium
         ∘ Potassium
         ∘ Calcium
         ∘ Chloride
         ∘ Magnesium
      ∘ Clotting
         ∘ Fibrinogen
         ∘ Quick Time / Prothrombin Time (TP)
         ∘ Cephalin Time / Activated Partial Thromboplastin Time (aPTT)
         ∘ International Normalized ratio (INR)
      ∘ Immunology
         ∘ Immunoglobulin G (IgG)
         ∘ Immunoglobulin A (IgA)
         ∘ Immunoglobulin M (IgM)
      ∘ Hepatic enzymes
         ∘ Aspartate aminotransferase (AST / GOT)
         ∘ Alanine aminotransferase (ALT / GPT)
         ∘ Alkaline phosphatase
         ∘ Gamma-glutamyl transferase (GGT)
   - Collect biological sample after procedure. It will be done at the same time as the extraction blood for analysis.
   - Take vital signs - Post LVPE
      ∘ Systolic and diastolic blood pressure
      ∘ Heart rate
      ∘ Respiratory rate
      ∘ Temperature
      ∘ Pulse oximetry to measure partial oxygen saturation

Data will also be collected from the TPE process once it has been completed.

It will be evaluated and collected all incidents and adverse events that may occur once finished the procedure either due to a problem with the machine, due to human error, due to problems with medication and treatment or for any other reason. Any medications will also be noted administered to the patient to resolve said adverse event.

### Internist

The internist physician will evaluate the patient at the end of treatment and will give them the medication, if necessary, until the next visit.

Also the internist physician will perform the medical report of the TPE. If the TPE number 6 (or the final TPE of the established guideline) has not been completed, the TPE treatment will be continued according to AMBAR standards or as prescribed by the internist physician. In the event that TPE number 6 (or the final TPE of the established pattern) has been completed, a second neurology visit will take place (TPE follow-up visit).

### Administration

From the center, a control call will be made to the patient, if possible on the same day in the afternoon or as a daily follow-up.

### Follow-up phase - Therapeutic plasma exchange (TPE)

The aim of the tracking phase is to assess the tolerance and efficacy of the treatment in order to decide on the continuation of it and switch to the LVPE phase. Likewise, follow-up visits will be used to collect clinical and biological information on AMBAR treatment.

To do this, the internist physician may, at his discretion, request the complementary visits described below to obtain additional information and, finally, a second Neurology/Medicine visit will be made to make a decision about the continuity of AMBAR treatment.

### Complementary TPE follow-up visit

Neuropsychological and functional assessment A neuropsychological exploration will be carried out by a qualified professional.

Nursing
- Obtaining nursing data (weight, height, body mass index, vital signs, electrocardiogram [ECG], tanita and vicorder)
- Perform blood tests
   ∘ Asking if the patient is fasting. This is a relevant question to biological samples obtained in the visit.
   ∘ Hematology

   - Complete blood count
      ∘ Red blood cells
      ∘ Hematocrit
      ∘ Hemoglobin
      ∘ Mean corpuscular volume (MCV)
      ∘ Mean corpuscular hemoglobin (HCM)
      ∘ Leukocyte
      ∘ Neutrophils
      ∘ Lymphocytes
      ∘ Monocytes
      ∘ Eosinophils
      ∘ Basophils
      ∘ Platelets
   - Erythrocyte sedimentation rate (ESR)
      ∘ Biochemistry
         ∘ Ferritin
         ∘ Total proteins
         ∘ Albumin
         ∘ Globulin
         ∘ Glycemia
         ∘ Urea
         ∘ Creatinine
         ∘ Sodium
         ∘ Potassium
         ∘ Calcium
         ∘ Chloride
         ∘ Magnesium
      ∘ Clotting
         ∘ Fibrinogen
         ∘ Quick Time / Prothrombin Time (TP)
         ∘ Cephalin Time / Activated Partial Thromboplastin Time (aPTT)
         ∘ International Normalized ratio (INR)
      ∘ Immunology
         ∘ Immunoglobulin G (IgG)
         ∘ Immunoglobulin A (IgA)
         ∘ Immunoglobulin M (IgM)
         ∘ Anti-thyroid antibodies
         ∘ Human immunodeficiency virus (HIV) serology
         ∘ Hepatitis C virus (HCV) serology
      ∘ Hepatic enzymes
         ∘ Aspartate aminotransferase (AST / GOT)
         ∘ Alanine aminotransferase (ALT / GPT)
         ∘ Alkaline phosphatase
         ∘ Gamma-glutamyl transferase (GGT)
      ∘ Thyroid tests
         ∘ T3
         ∘ T4
         ∘ free T4
         ∘ TSH
      ∘ Urine
         ∘ Sediment

### Second Neurology/Internal Medicine visit (TPE follow-up visit)

- Assess all the information available about the patient generated during the visits complementary follow-up (Neuropsychology and analytics)

Note: If levels of Neurology/Internal Medicine are detected during follow-up visits to Neurology/Internal Medicine, endogenous immunoglobulins below the lower limit of normal, the physician may decide the administration of intravenous immunoglobulin to normalize these levels.
- Assess previous and current medication. Readjust current treatment regimen based on drug and dosage
- Assess whether or not to continue treatment:
   ∘ in case of termination of the treatment, describe the reason and write a final report of all treatment received by the patient that will be delivered to the referring internist physician, the patient and / or family member / tutor.
   ∘ in case of continuation, schedule the LVPE treatment and write a follow-up report to be delivered to the referring internist physician, the patient and / or family member / tutor.

### Application phase - Low volume plasma exchange (LVPE)

### Before LVPE

### Nursing

Before any low-volume plasma exchange (LVPE) procedure, the nurse practitioner will scan the patient to verify that the patient can receive treatment. This exploration can be just before the treatment or a maximum of 48 hours before. This exploration will consist of:
- Asking if the patient is fasting. Treatment can be received whether the patient is in fast or not. This question is relevant for the biological samples obtained from the visit.
- Collect the weight, height and volume, calculated by the apheresis machine. In order to calculate the amount of plasma to be exchanged.
- Analytical - Pre LVPE
   ∘ Hematology

   - Complete blood count
      ∘ Red blood cells
      ∘ Hematocrit
      ∘ Hemoglobin
      ∘ Mean corpuscular volume (MCV)
      ∘ Mean corpuscular hemoglobin (HCM)
      ∘ Leukocyte
      ∘ Neutrophils
      ∘ Lymphocytes
      ∘ Monocytes
      ∘ Eosinophils
      ∘ Basophils
      ∘ Platelets
   - Erythrocyte sedimentation rate (ESR)
      ∘ Biochemistry
         ∘ Total proteins
         ∘ Albumin
         ∘ Globulin
         ∘ Glycemia
         ∘ Urea
         ∘ Creatinine
         ∘ Sodium
         ∘ Potassium
         ∘ Calcium
         ∘ Chloride
         ∘ Magnesium
      ∘ Clotting
         ∘ Fibrinogen
         ∘ Quick Time / Prothrombin Time (TP)
         ∘ Cephalin Time / Activated Partial Thromboplastin Time (aPTT)
         ∘ International Normalized ratio (INR)
      ∘ Immunology
         ∘ Immunoglobulin G (IgG)
         ∘ Immunoglobulin A (IgA)
         ∘ Immunoglobulin M (IgM)
      ∘ Hepatic enzymes
         ∘ Aspartate aminotransferase (AST / GOT)
         ∘ Alanine aminotransferase (ALT / GPT)
         ∘ Alkaline phosphatase ∘ Gamma-glutamyl transferase (GGT)
      ∘ COVID-19
         ∘ PCR SARS-COV-2
      ∘ Urine
         ∘ Sediment
   - Collect biological sample before procedure. It will be done at the same time as the extraction blood for analysis.
   - Perform an electrocardiogram (ECG)
   - Take vital signs - Pre LVPE
      ∘ Systolic and diastolic blood pressure
      ∘ Heart rate
      ∘ Respiratory rate
      ∘ Temperature
      ∘ Pulse oximetry to measure partial oxygen saturation

### internist

Then, the internist physician will make an assessment of the patient to determine whether or not the patient can receive the prescribed treatment. To do this, it must:
- Take an anamnesis of recent processes
- Review the exploration made by the nurse
- Assess possible new treatments started since the last visit

### Start the procedure

Once the internist physician concludes that the patient is suitable to receive the treatment, the internist physician will place the peripheral venous line to be able to connect the patient to the LVPE machine.

The treatment following the AMBAR standard consists of 1 LVPE procedures with replacement of 40 g of Albutein^{®} 20 % or Plasbumin^{®} 20 %. It will be carried out once a month during the time prescribed by the internist physician. This guideline can be modified according to medical criteria. Each procedure will replace between 690-880 ml of plasma, depending on the weight of the patient.

It will be noted on the data collection screens whether the procedure has started or not (and the reason for the which has not started), the time, in addition to all the information regarding the machine, solutions and material used for LVPE.

The LVPE procedure will be performed by the nurse under the supervision of the internist physician.

In the event that the internist physician considers that the patient is unfit to receive treatment, they will go directly to the second neurology visit ( LVPE follow-up visit).

In the event that any situation occurs that requires a medical emergency to the patient, the established protocol for this will be followed.

### During the LVPE

### Nursing

During the LVPE, the nurse practitioner will do an exploration of the patient to verify that the patient can continue receiving treatment. This exploration will consist of:
- Asking if the patient is fasting. Treatment can be continued whether the patient is in fast or not. This question is relevant for the biological samples obtained from the visit.
- Perform an analysis - During LVPE will be optional at the medical discretion and more can be done times depending on the patient's condition.
   ∘ Hematology
      ∘ Hemoglobin
   ∘ Biochemistry
      ∘ Glycemia
      ∘ Sodium
      ∘ Potassium
      ∘ Calcium
      ∘ Chloride
      ∘ Magnesium
   ∘ Acid/base equilibrium Clotting
- Collect vital signs. Three time during LVPE.
   ∘ Systolic and diastolic blood pressure
   ∘ Heart rate
   ∘ Respiratory rate
   ∘ Temperature
   ∘ Pulse oximetry to measure partial oxygen saturation

Data will be collected related with the estimated blood volume of the patient calculated by the LVPE machine. Also, data will be collected related to the LVPE process every 30 minutes.

To avoid potential adverse events related to hypovolaemia / hypotension, up to a maximum of 500 ml of saline solution may be infused at any time during the procedure.

It will be evaluated and collected all incidents and adverse events that may occur during the procedure either due to a problem with the machine, due to human error, due to problems with the medication or, for treatment or for any other reason. Any medications will also be noted administered to the patient to resolve said adverse event.

### After the LVPE

### Nursing

Once the procedure is finished, the end time will be noted, as well as the duration of the procedure. It is understood as the duration from when the patient enters the center until he leaves it. It will also be collected if the procedure has been completed or not (and the reasons why it has not been completed).

The nurse will do an exploration to the patient to verify the patient's condition after receive treatment. This exploration will consist of:
- Asking if the patient is fasting. This is a relevant question to biological samples obtained in the visit.
- Analytical - Post LVPE
   ∘ Hematology

   - Complete blood count
      ∘ Red blood cells
      ∘ Hematocrit
      ∘ Hemoglobin
      ∘ Mean corpuscular volume (MCV)
      ∘ Mean corpuscular hemoglobin (HCM)
      ∘ Leukocyte
      ∘ Neutrophils
      ∘ Lymphocytes
      ∘ Monocytes
      ∘ Eosinophils
      ∘ Basophils
      ∘ Platelets
   - Erythrocyte sedimentation rate (ESR)
      ∘ Biochemistry
         ∘ Total proteins
         ∘ Albumin
         ∘ Globulin
         ∘ Glycemia
         ∘ Urea
         ∘ Creatinine
         ∘ Sodium
         ∘ Potassium
         ∘ Calcium
         ∘ Chloride
         ∘ Magnesium
      ∘ Clotting
         ∘ Fibrinogen
         ∘ Quick Time / Prothrombin Time (TP)
         ∘ Cephalin Time / Activated Partial Thromboplastin Time (aPTT)
         ∘ International Normalized ratio (INR)
      ∘ Immunology
         ∘ Immunoglobulin G (IgG)
         ∘ Immunoglobulin A (IgA)
         ∘ Immunoglobulin M (IgM)
      ∘ Hepatic enzymes
         ∘ Aspartate aminotransferase (AST / GOT)
         ∘ Alanine aminotransferase (ALT / GPT)
         ∘ Alkaline phosphatase
         ∘ Gamma-glutamyl transferase (GGT)
   - Collect biological sample after procedure. It will be done at the same time as the extraction blood for analysis.
   - Take vital signs - Post LVPE
      ∘ Systolic and diastolic blood pressure
      ∘ Heart rate
      ∘ Respiratory rate
      ∘ Temperature
      ∘ Pulse oximetry to measure partial oxygen saturation

Data will also be collected from the LVPE process once it has been completed.

It will be evaluated and collected all incidents and adverse events that may occur once finished the procedure either due to a problem with the machine, due to human error, due to problems with medication and treatment or for any other reason. Any medications will also be noted administered to the patient to resolve said adverse event.

### Internist

The internist physician will evaluate the patient at the end of treatment and will give them the medication, if necessary, until the next visit.

Also the internist physician will perform the medical report of the LVPE. If the LVPE number 3 (or if no more than 3 months are after the last neurological visit) has not been completed, the LVPE treatment will be continued according to AMBAR standards or as prescribed by the internist physician. In the event that LVPE number 3 (or if more than 3 months are after the last neurological visit) has been completed, a second neurology visit will take place (LVPE follow-up visit).

### Administration

From the center, a control call will be made to the patient, if possible on the same day in the afternoon or daily following.

### Follow-up Phase - Low volume plasma exchange (LVPE)

The objective of the follow-up phase is to assess the tolerance and efficacy of AMBAR medical treatment for decide on the continuation of it during the LVPE phase. Likewise, follow-up visits will be used to collect clinical and biological information on AMBAR treatment.

To do this, the internist physician may, at his discretion, request the complementary visits described below to obtain additional information and to finalize a Second Neurology/Medicine visit will be made to make a decision about the continuity of AMBAR treatment. Follow-up visits during the LVPE phase will be carried out approximately every 3 months.

### Complementary LVPE follow-up visit

### Neuropsychological and functional assessment

A neuropsychological exploration will be carried out by a qualified professional.

### Nursing

- Obtaining nursing data (weight, height, body mass index, vital signs, electrocardiogram [ECG], tanita and vicorder)
- Perform blood tests
   ∘ Asking if the patient is fasting. This is a relevant question to biological samples obtained in the visit.
   ∘ Hematology

   - Complete blood count
      ∘ Red blood cells
      ∘ Hematocrit
      ∘ Hemoglobin
      ∘ Mean corpuscular volume (MCV)
      ∘ Mean corpuscular hemoglobin (HCM)
      ∘ Leukocyte
      ∘ Neutrophils
      ∘ Lymphocytes
      ∘ Monocytes
      ∘ Eosinophils
      ∘ Basophils
      ∘ Platelets
   - Erythrocyte sedimentation rate (ESR)
      ∘ Biochemistry
         ∘ Albumin
         ∘ Globulin
         ∘ Glycemia
         ∘ Urea
         ∘ Creatinine
         ∘ Sodium
         ∘ Potassium
         ∘ Calcium
         ∘ Chloride
         ∘ Magnesium
      ∘ Clotting
         ∘ Fibrinogen
         ∘ Quick Time / Prothrombin Time (TP)
         ∘ Cephalin Time / Activated Partial Thromboplastin Time (aPTT)
         ∘ International Normalized ratio (INR)
      ∘ Immunology
         ∘ Immunoglobulin G (IgG)
         ∘ Immunoglobulin A (IgA)
         ∘ Immunoglobulin M (IgM)
         ∘ Anti-thyroid antibodies
         ∘ Human immunodeficiency virus (HIV) serology
         ∘ Hepatitis C virus (HCV) serology
      ∘ Hepatic enzymes
         ∘ Aspartate aminotransferase (AST / GOT)
         ∘ Alanine aminotransferase (ALT / GPT)
         ∘ Alkaline phosphatase
         ∘ Gamma-glutamyl transferase (GGT)
      ∘ Thyroid tests
         ∘ T3
         ∘ T4
         ∘ free T4
         ∘ TSH
      ∘ Urine
         ∘ Sediment

### Second Neurology/internal Medicine visit (LVPE follow-up visit)

- Assess all the information available about the patient generated during the visits complementary follow-up (Neuropsychology and analytics)

Note: If levels of Neurology/Internal Medicine are detected during follow-up visits to Neurology/Internal Medicine, endogenous immunoglobulins below the lower limit of normal, the physician may decide the administration of intravenous immunoglobulin to normalize these levels.
- Assess previous and current medication. Readjust current treatment regimen based on drug and dosage.
- Assess whether or not to continue treatment:
   ∘ in case of termination of the treatment, describe the reason and write a final report of all treatment received by the patient that will be delivered to the referring internist physician, the patient and / or family member / tutor.
   ∘ in case of continuation, schedule the LVPE treatment and write a follow-up report to be delivered to the referring internist physician, the patient and / or family member / tutor.

### EXAMPLES

### Example 1: Safety and effectiveness of plasma exchange with albumin replacement in Alzheimer's Disease

99 patients suffering from AD were selected and were evaluated for a treatment that included 6 biweekly therapeutic plasma exchange (TPE) intensive period followed, at least, by 12 monthly low volume plasma exchange (LVPE) maintenance period. In order to evaluate treatment progress and safety, all patients underwent neurological, neuropsychological and medical evaluations at baseline, at 12 weeks and every 6 months.

**Table 1. Gender and age distribution of the 40 patients that completed the treatment.**

| | **Male** | | **Female** |
|---|---|---|---|
| **Gender distribution** | 16 (40 %) | | 24 (60 %) |
| **Age** | | 71,4 +/- 7,1 years | |

Of the 99 subjects, 76 were eligible to start treatment and 40 have completed it up to the date of this analysis (the rest are ongoing). In the group of 40 people that completed the treatment, the age of the patients was around 71 years and it was roughly equal between both genders (Table 1). The initial mean Mini Mental State Examination (MMSE) of said group was 19,0 +/- 5.2 (Cl₉₅: 17.4-20.6), while at the end of the treatment the mean was of 18.0 +/- 6.2 (Cl₉₅: 16.4-19.6) indicating an average stabilization taking into account that AD is a highly deteriorating illness that normally decreases MMSE scores at a much faster pace that can reach up to 5-6 points per year (Mittal S, Singh J, Pathania M, Goel A (2017) Up in Arms against Alzheimer's Disease, J Neurol Exp Neural Sci 2017: JNNS-127). A closest observation at this average behaviour shows that 34 of the 40 patients (85 %) showed an improvement or at least stability in MMSE (variation <= 3 points).

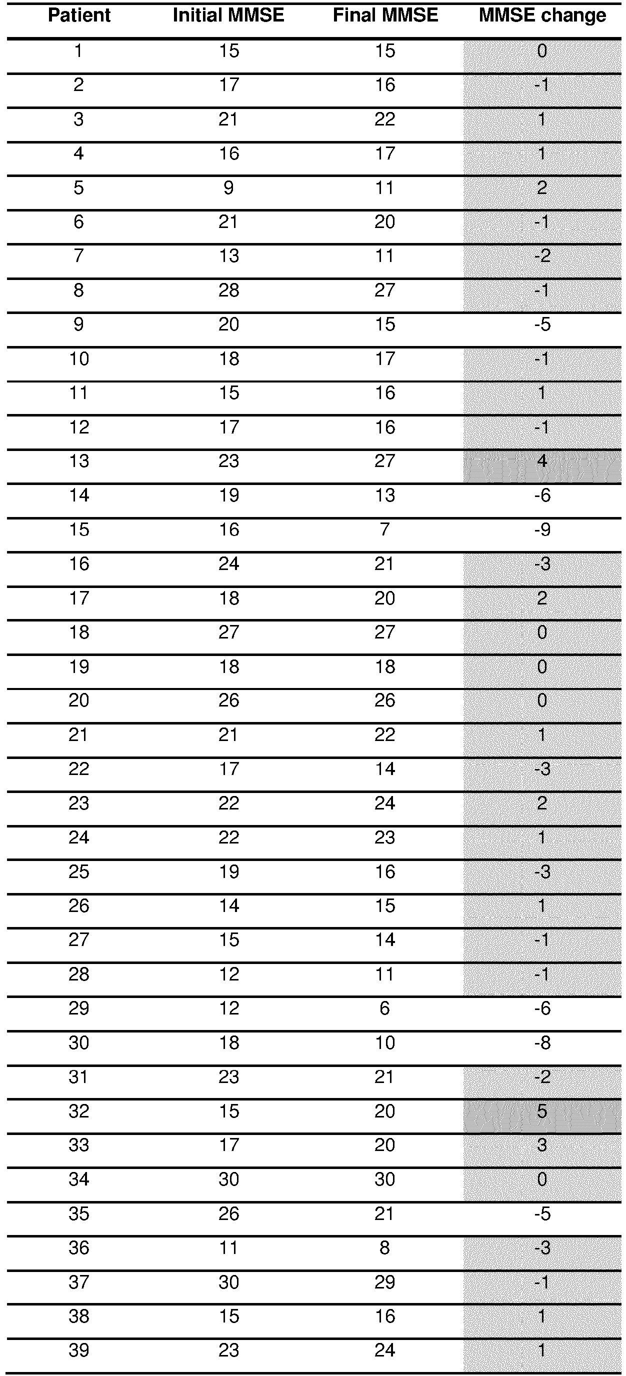

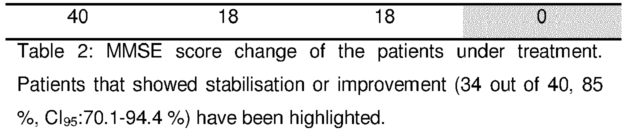

The total number of therapeutic plasma exchange (TPE) performed was 240, of which 224 (93.3 %, Cl₉₅:89.0-96.0 %) were uneventful. The most frequent adverse effects were in fact very low in number: hypotension (4 cases), discomfort (4 cases), syncope episodes (2 cases), hypogammaglobulinemia (2 cases) and paresthesia (1 case). However, none of said adverse effects required discontinuation of the treatment program by the patient, thus showing the safety of the treatment, along with the efficacy previously shown, demonstrating that plasma exchange with albumin replacement in AD patients is safe and effective for clinical practice, and shows a trend in cognitive stabilization even in short term exposures and limited sample sizes.

## Claims

1. Composition comprising human albumin at a concentration between 5 % (w/v) and 25 % (w/v) for the treatment of a cognitive impairment in a patient in need thereof, wherein said treatment comprises a combination of conventional therapeutic plasma exchange (TPE) and low-volume plasma exchange (LVPE), and wherein said patient has a Mini Mental State Examination (MMSE) greater or equal to 15.

2. Composition for use, according to claim 1, **characterized in that** said LVPE regime involves blood volumes between 600 mL - 900 mL.

3. Composition for use, according to claim 1 or 2, **characterized in that** said TPE regime involves blood volumes between 2000 to 3000 mL.

4. Composition for use, according to any of the preceding claims, **characterized in that** before the treatment regime of LVPE previous rounds of TPE are carried out.

5. Composition for use, according to claim 4, **characterized in that** said TPE regime can be conducted at a frequency of 1 TPE per week.

6. Composition for use, according to claim 4 or 5, **characterized in that** said TPE regime is conducted at least during 6 weeks.

7. Composition for use, according to claims 4 to 6, **characterized in that** in said TPE regime the plasma is replaced with albumin at a concentration between 4 % (w/v) and 6 % (w/v), preferably at 5 % (w/v).

8. Composition for use, according to any of the preceding claims, **characterized in that** each subsequent round of LVPE is conducted 10-45 days after the previous round.

9. Composition for use, according to any of the preceding claims, **characterized in that** said LVPE is conducted at a frequency of 1 LVPE per month.

10. Composition for use, according to claim 9, **characterized in that** said monthly LVPE is conducted at least during 12 months.

11. Composition for use, according to any of the preceding claims, **characterized in that** between 20 g and 50 g of albumin are used for replacement in each round of LVPE.

12. Composition for use, according to claim 11, **characterized in that** 40 g of albumin are used for replacement in each round of LVPE.

13. Composition for use, according to claims 8 to 12, **characterized in that** in said LVPE regime the plasma is replaced with albumin at a concentration between 18 % (w/v) and 25 % (w/v), preferably at 20 % (w/v).

14. Composition for use, according to any of the preceding claims, **characterized in that** said cognitive impairment is selected from the list comprising Alzheimer's Disease (AD), Lewy Body Dementia (LBD), Parkinson's Disease (PD), and Mild Cognitive impairment (MCI).

15. Composition for use, according to any of the preceding claims, **characterized in that** said cognitive impairment is Mild Cognitive impairment (MCI).
